(19) 

Europäisches
Patentamt

European
Patent Office

Office européen
des brevets

(11)  **EP 4 717 271 A1**

(12)  **EUROPEAN PATENT APPLICATION**
published in accordance with Art. 153(4) EPC

(43) Date of publication:
    **01.04.2026  Bulletin 2026/14**

(21) Application number: **24842274.3**

(22) Date of filing: **11.07.2024**

(51) International Patent Classification (IPC):
    *A61K 38/39* (2006.01)    *C07K 14/78* (2006.01)
    *A61P 9/00* (2006.01)    *A61P 9/12* (2006.01)
    *A61K 8/65* (2006.01)

(52) Cooperative Patent Classification (CPC):
    **A61K 8/65; A61K 38/39; A61K 45/06; A61K 48/00;
    A61L 15/32; A61L 15/44; A61L 26/00; A61L 31/04;
    A61L 31/16; A61P 3/10; A61P 7/02; A61P 9/00;
    A61P 9/04; A61P 9/06; A61P 9/10;**          (Cont.)

(86) International application number:
    **PCT/CN2024/104947**

(87) International publication number:
    **WO 2025/016280 (23.01.2025 Gazette 2025/04)**

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **GE KH MA MD TN**

(30) Priority:  **19.07.2023   CN 202310890232**

(71) Applicant: **Shanxi Jinbo Bio-Pharmaceutical Co.,
    Ltd.
    Shanxi 030032 (CN)**

(72) Inventors:
    • **YANG, Xia**
      **Taiyuan, Shanxi 030032 (CN)**
    • **PEI, Yu**
      **Taiyuan, Shanxi 030032 (CN)**
    • **YU, Yufeng**
      **Taiyuan, Shanxi 030032 (CN)**
    • **HE, Zhenrui**
      **Taiyuan, Shanxi 030032 (CN)**

(74) Representative: **Cabinet Becker et Associés
    25, rue Louis le Grand
    75002 Paris (FR)**

Remarks:
    The complete document including Reference
    Table(s) and the Sequence Listing(s) can be
    downloaded from the EPO website

(54)  **164.88-DEGREE RECOMBINANT HUMANIZED TYPE III COLLAGEN FOR VASCULAR REPAIR**

(57)     Provided is 164.88-degree recombinant humanized type III collagen for vascular repair. The present invention relates to a use of collagen in preparation of a medical device or a drug or a kit. The medical device or drug or kit is used for prevention and/or treatment of cardiovascular diseases or for vascular injury repair. The collagen comprises n repeated units, and each repeated unit comprises a sequence as shown in SEQ ID NO: 1. The collagen can be used for treating or preventing cardiovascular diseases.

EP 4 717 271 A1

(52) Cooperative Patent Classification (CPC): (Cont.)
**A61P 9/12; A61P 9/14; A61P 17/02; A61P 17/16;**
**C07K 14/78; C12N 5/06; G01N 33/68**

## Description

This application claims the priority right of the Chinese patent application No. 202310890232.8 filed on July 19, 2023, and entitled "164.88-DEGREE RECOMBINANT HUMANIZED TYPE III COLLAGEN FOR VASCULAR REPAIR". This patent application is incorporated herein by reference in its entirety.

Technical Field

The present disclosure relates to the field of collagen applications, in particular to the application of 164.88-degree recombinant type III humanized collagen for vascular repair and reconstruction or antihypertension.

Background Art

Collagen is the main structural protein of human tissues and organs, accounting for approximately 30-40% of the total protein in the body, mainly located in skin, bone and muscle tissues. As a major component of the extracellular matrix, collagen may meet the requirements of an excellent biomaterial, such as favorable biocompatibility, appropriate mechanical strength, moderate flexibility, and cell adhesion activity. Thus, it is a structural protein with biotechnological value. To date, at least 28 types of collagens have been reported, which are located in different positions in the body and have been named from type I to type XXVIII according to the time of discovery. The distinctive feature of collagen is a right-handed triple helix domain, i.e., a protein with a repeating Gly-X-Y triplet, in which proline and lysine usually appear at the X and Y positions. However, concerns about purity, disease transmission, and reproducibility of animal-derived collagen limit its application and require alternative recombinant sources.

With advances in genetic engineering and synthetic biology, recombinant collagen proteins with specific structures and functions can be developed and show potential as starting materials for surgical implants and matrices for regenerative medicine. Generally speaking, a recombinant collagen can be prepared by transcribing specific gene fragments and expressed in yeast, bacteria, or animal cells, leading to consistent formulation and yield and high productivity. In order to be biocompatible with the human body, a human collagen gene is mainly selected to encode a specific type of human collagen to produce predictable and reliable recombinant collagen, which has wide medical application prospects. In March 2021, China's National Medical Products Administration (NMPA) issued standards and guidelines for the definition of recombinant collagen, in which a recombinant humanized or human-derived collagen is defined as a full-length amino acid sequence encoded by a specific type of human collagen gene or a fragment thereof with function, or a combination containing functional fragments of human collagen.

Recombinant humanized type III collagen (RhCOL III) was developed by Shanxi Jinbo Biomedical Co., Ltd. This technological research is based on the original gene sequence of human type III collagen, the segments with strong water solubility and high biological activity is optimized and selected for codon optimization and splicing recombination, resulting in a brand-new recombinant human collagen sequence. Experiments confirm that this collagen has a high expression level, good water solubility and high biological activity, and its performance is better than that of natural human collagen. Studies have shown that RhCOL III has the efficacy of anti-photoaging, promoting wound repair, promoting proliferation, and so on. The antihypertensive activity of RhCOL III has not been reported.

There is a need to further expand the application of a recombinant humanized type III collagen in the art.

Summary of the Disclosure

The inventors have previously discovered a recombinant type III collagen with high cell adhesion properties. It has advantages such as high stability in aqueous solution, ease of preparation and purification (see CN 201811438582.6, which is incorporated herein by reference). In order to expand the application of the recombinant type III collagen, the inventors have carried out a lot of biological activity studies. Surprisingly, the inventors have found that the recombinant type III collagen can have vascular injury repair activity and can be used for preventing and/or treating cardiovascular diseases. The preparation of this protein refers to the inventor's patent 201811438582.6, in which it has been mentioned that the crystal structure of this protein has been included in the PDB database under accession numbers 6A0A and 6A0C. The analysis of the protein structure can be found in Hua C, Zhu Y, Xu W, Ye S, Zhang R, Lu L, Jiang S. Characterization by high-resolution crystal structure analysis of a triple-helix region of human collagen type III with potent cell adhesion activity. Biochem Biophys Res Commun. 2019 Jan 22;508(4):1018-1023. doi: 10.1016/j.bbrc.2018.12.018. Epub 2018 Dec 11. PMID: 30545625; PMCID: PMC7092849, which reveals that this protein has a 164.88-degree curved structure.

In the first aspect, the present application provides use of a collagen (also referred to as a polypeptide or a protein), a fusion protein comprising the collagen, a nucleic acid encoding the collagen or the fusion protein, a vector comprising the nucleic acid, a host cell comprising the vector, or a composition comprising the collagen, the fusion protein, the nucleic acid, the vector and/or the host cell for the manufacture of a medical device or a medicament or a drug-device combination product or a kit for preventing and/or treating a cardiovascular disease or for the repair of a vascular injury or for preventing and/or treating a vascular injury in a subject, wherein the collagen comprises n repeat units, wherein the repeat unit comprises:

(1) a sequence set forth in SEQ ID NO: 1 (GERGAPGFRGPAGPNGIPGEKGPAGERERGAP);
(2) a sequence having at least 80%, 85%, 90%, 91%, 92%, 93%, 94%, 95%, 96%, 97%, 98%, or 99% identity to the sequence set forth in SEQ ID NO. 1; or
(3) a sequence in which a substitution, an addition, a deletion and/or an insertion of 1 to 5 amino acid residues is introduced to the sequence set forth in SEQ ID NO. 1;

wherein n is an integer greater than or equal to 1.

In one embodiment, the collagen is a recombinant type III humanized collagen, preferably 164.88-degree recombinant type III humanized collagen. Preferably, the recombinant type III humanized collagen is in a trimeric form.

In one embodiment, the subject has an ultraviolet photoaging-induced skin injury or a diabetic infected wound.

In one embodiment, the collagen has an anti-endothelial cell injury effect.

In one embodiment, the fusion protein comprises the collagen and a protein for promoting secretion, isolation and/or purification of the collagen.

In one embodiment, the protein is selected from an enzyme cleavage site sequence, a signal peptide and a purification tag sequence.

In one embodiment, the enzyme cleavage site sequence is a TEV protease cleavage site sequence.

In one embodiment, the protein tag sequence is His tag, GST tag, MBP tag, SUMO tag, Cytiva Protein Select tag or NusA tag.

In one embodiment, the collagen is linked to the protein directly or through a linker, wherein the linker is a flexible linker, such as $(G)_a$ or $(GGGGS)_b$, wherein a and b are each independently an integer from 1 to 10 or from 1 to 5 or from 1 to 3.

In one embodiment, the medical device or the medicament or the drug-device combination product or the kit is also used for preventing and/or treating an ultraviolet photoaging-induced skin injury or a diabetic infected wound.

In one embodiment, the medical device is a gel, a dressing, an invasive device, or an implantable device.

In one embodiment, n is an integer from 1 to 32. In one embodiment, when n is an integer greater than or equal to 2, the repeat sequences are directly linked to each other. In one embodiment, n is 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31.

In one embodiment, the collagen comprises the sequence set forth in SEQ ID NO. 2 (GPPGPCCGGG) or does not comprise the sequence set forth in SEQ ID NO. 2 (GPPGPCCGGG).

In one embodiment, the collagen comprises:

a) an amino acid sequence of SEQ ID NO. 3;
b) an amino acid sequence having at least 80%, 85%, 90%, 91%, 92%, 93%, 94%, 95%, 96%, 97%, 98%, or 99% identity to the amino acid sequence of SEQ ID NO. 3 while retaining the anti-endothelial cell injury effect of the amino acid sequence of SEQ ID NO. 3; or
c) an amino acid sequence in which 1 to 80 (such as 1 to 70, 1 to 60, 1 to 50, 1 to 40, 1 to 30, 1 to 10, 1 to 8 or 1 to 5) amino acid residues are added, substituted, deleted or inserted into the amino acid sequence of SEQ ID NO. 3 while retaining the anti-endothelial cell injury effect of the amino acid sequence of SEQ ID NO. 3.

In one embodiment, the amino acid sequence of SEQ ID NO. 3 is GERGAPGFRGPAGPNGIPGEKGPAGER GAPGERGAPGFRGPAGPNGIPGEKGP AGERGAPGERGAPGFRGPAGPNGIPGEKGPAGERGAPGERGAPGFRGP AGPNGIPGEK GPAGERGAPGERGAPGFRGPAGPNGIPGEKGPAGERGAPGERGAPGFRGPAGPNGIPG EKGPAG ERGAPGERGAPGFRGPAGPNGIPGEKGPAGERGAPGERGAPGFRGPAGPNGI PGEKGPAGERGAPGERGAPGFR GPAGPNGIPGEKGPAGERGAPGERGAPGFRGPAGPN IPGEKGPAGERGAPGERGAPGFRGPAGPNGIPGEKGPA GERGAPGERGAPGFRGPAG PNGIPGEKGPAGERGAPGERGAPGFRGPAGPNGIPGEKGPAGERGAPGERGAPGF RGP AGPNGIPGEKGPAGERGAPGERGAPGFRGPAGPNGIPGEKGPAGERGAPGERGAPGFR GPAGPNGIPGEKG-PAGERGAP. In the present disclosure, a collagen having the amino acid sequence of SEQ ID NO: 3 is also referred to as RhCOL III.

In one embodiment, the cardiovascular disease is a cardiovascular disease associated with an endothelial cell injury. In one embodiment, the endothelial cell injury is an endothelial cell injury induced by angiotensin II or ROS, and/or the vascular injury is a vascular injury induced by angiotensin II or ROS. In one embodiment, the vascular injury is a vascular endothelial injury, preferably a diabetic vascular endothelial injury.

In one embodiment, the cardiovascular disease is selected from arteriosclerosis, atherosclerosis, hypertension, coronary heart disease, peripheral vascular disease, stroke, heart failure, arrhythmia, cerebrovascular disease, atrial fibrillation, and thrombotic disease. In one embodiment, the hypertension is spontaneous hypertension.

In one embodiment, the medical device or the medicament or the kit is a medical device or a medicament or a kit for lowering blood pressure.

In one embodiment, the composition comprises a lipid-regulating agent and/or an antihypertensive agent, such as statins, an angiotensin converting enzyme inhibitor, a calcium channel blocker and/or a beta receptor blocker.

In the second aspect, the present application provides a pharmaceutical composition or a medical device or a drug-device combination product. In one embodiment, the pharmaceutical composition comprises the collagen, the fusion protein comprising the collagen, the nucleic acid encoding the collagen or the fusion protein, the vector comprising the nucleic acid, the host cell comprising the vector in the first aspect hereabove, and an agent for preventing and/or treating a cardiovascular disease or for the repair of a vascular injury. In one embodiment, the agent is a lipid-regulating agent and/or an antihypertensive agent, such as statins, an angiotensin converting enzyme inhibitor, a calcium channel blocker and/or a beta receptor blocker. In another embodiment, the medical device or the drug-device combination product comprises the collagen, the fusion protein comprising the collagen, the nucleic acid encoding the collagen or the fusion protein, the vector comprising the nucleic acid, the host cell comprising the vector in the first aspect hereabove, and a material or device for the repair of a vascular injury. Preferably, the medical device is a gel, a dressing, an invasive device, or an implantable device. In one embodiment, the material or device is for use as a medical device.

In the third aspect, the present application provides a method of blocking or eliminating an angiotensin II-induced vascular endothelial cell injury *in vitro,* comprising a step of contacting the vascular endothelial cell with the collagen in the first aspect hereabove. In one embodiment, the vascular endothelial cell is an umbilical vein vascular endothelial cell, preferably a human umbilical vein vascular endothelial cell.

In the fourth aspect, the present application provides an *in vitro* method for (1) reducing angiotensin II-induced ROS generation in a cell and/or (2) alleviating microtubule injury in a cell, comprising a step of contacting cells with the collagen in the first aspect herein. In one embodiment, the cell is a vascular endothelial cell, preferably an umbilical vein vascular endothelial cell, preferably a human umbilical vein vascular endothelial cell. In one embodiment, the microtubule injury is Ang II-induced microtubule injury.

In the fifth aspect, the present application provides a method for preventing and/or treating a cardiovascular disease or for the repair of a vascular injury or for preventing and/or treating a vascular injury, comprising administering to a subject the collagen, the fusion protein comprising the collagen, the nucleic acid encoding the collagen or the fusion protein, the vector comprising the nucleic acid, the host cell comprising the vector, or the composition comprising the collagen, the fusion protein, the nucleic acid, the vector, and/or the host cell in the first aspect hereabove, or administering to a subject the pharmaceutical composition or the medical device or the drug-device combination product of the second aspect herein.

In one embodiment, the cardiovascular disease is a cardiovascular disease associated with an endothelial cell injury. In one embodiment, the endothelial cell injury is an endothelial cell injury induced by angiotensin II or ROS, and/or the vascular injury is a vascular injury induced by angiotensin II or ROS.

In one embodiment, the cardiovascular disease is selected from arteriosclerosis, atherosclerosis, hypertension, coronary heart disease, peripheral vascular disease, stroke, heart failure, arrhythmia, cerebrovascular disease, atrial fibrillation, and thrombotic disease.

In one embodiment, the composition comprises a lipid-regulating agent and/or an antihypertensive agent, such as statins, an angiotensin converting enzyme inhibitor, a calcium channel blocker and/or a beta receptor blocker.

In one aspect, a method of screening for an active ingredient for preventing and/or treating a cardiovascular disease or for the repair of a vascular injury in a subject is provided, comprising

(i) providing a collagen comprising n repeat units, wherein the repeat unit comprises:

(1) a sequence set forth in SEQ ID NO. 1;
(2) a sequence having at least at least 70%, at least 80%, at least 85%, at least 90%, at least 91%, at least 92%, at least 93%, at least 94%, at least 95%, at least 96%, at least 97%, at least 98%, or at least 99% identity to the sequence set forth in SEQ ID NO. 1; or
(3) a sequence in which a substitution, an addition, a deletion and/or an insertion of 1-5 amino acid resiudes is introduced to the sequence set forth in SEQ ID NO. 1;

wherein n is an integer greater than or equal to 1, preferably an integer ranging from 1 to 32, such as 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31, wherein when n is an integer greater than or equal to 2, the repeat sequences are directly linked to each other;
(ii) adding the collagen to an angiotensin II-treated HUVEC cell;
(iii) selecting the collagen having a property of (a) reducing ROS/or (b) alleviating microtubule injury as an active ingredient.

In one embodiment, the subject has an ultraviolet photoaging-induced skin injury or a diabetic infected wound.

In one embodiment, the collagen has an anti-endothelial cell injury effect.

In one embodiment, the active ingredient is further useful for preventing and/or treating an ultraviolet photoaging-induced skin injury or a diabetic infected wound.

In one embodiment, the cardiovascular disease is a cardiovascular disease associated with an endothelial cell injury, preferably, the endothelial cell injury is an endothelial cell injury induced by angiotensin II or ROS, and/or the vascular injury is a vascular injury induced by angiotensin II or ROS.

In one embodiment, the cardiovascular disease is selected from atherosclerosis, hypertension, coronary heart disease, peripheral vascular disease, stroke, heart failure, arrhythmia, cerebrovascular disease, atrial fibrillation, and thrombotic disease.

In one embodiment, the selected collagen comprises:

a) an amino acid sequence of SEQ ID NO. 3;

b) an amino acid sequence having at least 80%, at least 85%, at least 90%, at least 91%, at least 92%, at least 93%, at least 94%, at least 95%, at least 96%, at least 97%, at least 98%, or at least 99% identity to the amino acid sequence set forth in SEQ ID NO. 3; or

c) an amino acid sequence in which 1 to 80, such as 1 to 70, 1 to 60, 1 to 50, 1 to 40, 1 to 30, 1 to 10, 1 to 8 or 1 to 5 amino acid residues are added, substituted, deleted or inserted into the amino acid sequence of SEQ ID NO. 3.

In one embodiment, the active ingredient is used as a medical device or as a medicament or a drug-device combination product.

The advantages of this application include:

1. RhCOL III collagen does not affect the viability of HUVEC cells.
2. RhCOL III collagen significantly inhibits the production of ROS in HUVECs.
3. RhCOL III treatment of cells can significantly alleviate microtubule injury.
4. RhCOL III collagen can reduce blood pressure *in vivo,* as demonstrated in spontaneously hypertensive rats.

Description of the Drawings

Fig. 1 shows that RhCOL III interacts with HUVEC cells.

Fig. 2 shows the effect of RhCOL III on the viability of HUVECs. $**$ indicates $p < 0.05$, $***$ indicates $p < 0.001$, compared to the control group (Ang II induction group).

Fig. 3 shows detection of intracellular total ROS levels in HUVECs with the fluorescent probe DCFH-DA. $***$ indicates $p < 0.001$, compared to the control group (Ang II induction group).

Fig. 4 shows the effect of RhCOL III on the microtubule formation of HUVEC cells.

Fig. 5 shows the effect of RhCOL III on the blood pressure, weight, heart rate, and heart weight ratio of SHR. (A) systolic blood pressure, (B) heart rate, (C) body weight, and (D) heart weight ratio are measured. $*$ indicates $P<0.01$, $**$ indicates $p < 0.05$, $***$ indicates $p < 0.001$, compared to the control group.

Fig. 6 shows the effect of RhCOL III on the blood biochemical indicators of SHR. (A) Ang II, (B) LDH, (C) UA, (D) ALT, (E) CERA, (F) LAC. $***$ indicates $p < 0.001$, compared to the control group.

Detailed Description of the Disclosure

Further description is provided below to facilitate understanding of the present disclosure.

As used herein, the term "vascular endothelial cell (VEC)", also referred to as endothelial cell, means a layer of flattened mononuclear cells between the bloodstream and the blood vessel wall. VEC injury plays a very important role in the pathogenesis and pathological processes such as atherosclerotic heart disease, acute myocardial infarction, acute myocardial infarction no-reflow, various cerebrovascular diseases, and renal vascular diseases. When vascular endothelial cell experiments are performed, the cell model usually selected is "human umbilical vein endothelial cells" (abbreviated as HUVECs). The vascular endothelium covers the inner wall of the blood vessel and is in direct contact with the blood in the lumen of the blood vessel. Studies have shown that impaired vascular endothelial function and NO metabolism disorders are the initiating factors and central links in atherosclerosis (AS). An endothelial injury is the first step of macroscopic and microscopic vascular dysfunction in diabetic patients and is the cause of the high incidence of diabetic vascular complications such as atherosclerosis, nephropathy, retinopathy and neuropathy. As the main cell type in endothelial tissue, endothelial cells play a crucial role in regulating vascular structure and function by releasing vasoactive factors such as prostacyclin (PGI2), reactive oxygen species (ROS), endothelin-1 (ET-1), nitric oxide (NO), and angiotensin II (Ang II). After long-term exposure to a hyperglycemic environment, the vasoconstrictive and inflammatory factors (such as TNF - $\alpha$, Ang II, ROS, and ET-1) secreted by endothelial cells increase, and NO production and high-density lipoprotein (HDL) uptake decrease, resulting in a fragile vascular system, increased oxidative stress, and impaired endothelial repair, which is a state that promotes atherosclerosis.

As used herein, "angiotensin (Ang) II" is a particularly important vasoactive substance in the renin-Ang-aldosterone system (RAAS) and is an important contributor to various cardiovascular diseases. It has the function of contracting blood vessels and is mainly secreted by VECs and vascular smooth muscle cells. It leads to structural and functional injury of VECs through various mechanisms, produces significant adverse effects on the ultrastructure, barrier function and secretion function of VECs, and can also cause cell senescence and induce its apoptosis.

As used herein, the term "medical device" refers to an instrument, a device, an appliance, an *in vitro* diagnostic reagent and a calibrator, a material, and other similar or related articles that are directly or indirectly used in the human body. The collagen herein can achieve medical device application, and thus can be used as a medical device. In some embodiments, the collagen of the present disclosure exerts pharmacological effects and can be used as a medicament. In one embodiment, the collagen of the present disclosure is used as a drug-device combination product. A drug-device combination product can be classified as either a drug-led or device-led according to the main mechanism of action of collagen. The collagen of the present disclosure can be used as a medical device. For example, after collagen is injected, its triple helix structure self-assembles through intermolecular interactions to form a collagen fiber network. This network structure supports cells and tissues, physically connects damaged blood vessels, improves the mechanical properties and elasticity of blood vessels, and finally realizes vascular injury repair.

By "pharmaceutical composition" is meant that the composition comprises the collagen of the present disclosure in combination with at least one additional pharmaceutically acceptable carrier. The "pharmaceutically acceptable carrier" refers to a medium generally accepted in the art for the delivery of a biologically active agent to an animal, particularly a mammal, including adjuvants, excipients or vehicles such as diluents, preservatives, fillers, disintegrants, wetting agents, emulsifiers, suspending agents, sweetening agents, flavoring agents, perfuming agents, antibacterial agents, antifungal agents, lubricants and dispersing agents.

As used herein, "recombinant type III collagen" is the full-length amino acid sequence encoded by the human type III collagen gene or fragment thereof having function, or a combination containing functional fragments of human collagen. Herein, RhCOLIII is a plurality of repeat units which are human type III collagen fragments. The recombinant type III collagen described herein may comprise a small amount of non-native sequence at the N-terminus and/or C-terminus, for example, GPPGPCCGGG (SEQ ID NO. 2). The sequence of the repeating unit may be derived from human. The recombinant type III collagen described herein may have vascular endothelial cell repair function, or may block or eliminate Ang II-induced injury to vascular endothelial cells. In some embodiments, the collagen described herein is prepared synthetically. In some embodiments, the collagen described herein is prepared by expression in a prokaryote, such as *E. coli.* In some embodiments, the collagen described herein is prepared by expression in a eukaryote, for example a yeast cell, such as *Komagataella phaffii* cell. In a preferred embodiment, the collagen described herein is expressed by *E. coli.*

For example, recombinant type III collagen can comprise: a) an amino acid sequence of SEQ ID NO. 3; b) an amino acid sequence having at least 80%, at least 85%, at least 90%, at least 91%, at least 92%, at least 93%, at least 94%, at least 95%, at least 96%, at least 97%, at least 98%, or at least 99% identity to the amino acid sequence of SEQ ID NO. 3 while retaining the anti-endothelial cell injury effect of the amino acid sequence of SEQ ID NO. 3; or c) an amino acid sequence in which 1 to 80 amino acid residues are mutated (*e.g.,* added, substituted, deleted, or inserted) in the amino acid sequence of SEQ ID NO. 3 while retaining the anti-endothelial cell injury effect of the amino acid sequence of SEQ ID NO. 3. The number of mutations may be 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80.

As used herein, the repeat unit is a fragment derived from a naturally occurring protein, such as type III collagen. The recombinant type III collagen described herein can be formed by linking the repeating units in a certain number. In the present disclosure, the repeat sequence of SEQ ID NO. 1 used is GERGAPGFRGPAGPNGIPGEKGPAGERGAP (SEQ ID NO. 1). The collagen of the present disclosure may comprise a plurality of repeat sequences, wherein there is no linker between the repeat sequences. The repeat sequence can also be a variant of the sequence set forth in SEQ ID NO. 1, for example, a sequence having at least 80%, at least 85%, at least 90%, at least 91%, at least 92%, at least 93%, at least 94%, at least 95%, at least 96%, at least 97%, at least 98%, or at least 99% identity to the sequence set forth in SEQ ID NO. 1; or a sequence in which 1 to 5 (1, 2, 3, 4 or 5) substitutions, additions, deletions and/or insertions are introduced into the sequence set forth in SEQ ID NO. 1. The repeating number n of the repeating units can be an integer greater than or equal to 1. For example, n may be an integer from 1 to 32, such as 2, 3, 4, 5, 6, 7, 8, 9, 10, 11, 12, 13, 14, 15, 16, 17, 18, 19, 20, 21, 22, 23, 24, 25, 26, 27, 28, or 29.

As used herein, a host cell may be a eukaryotic cell, such as fungi and yeast, or a prokaryotic cell, such as enterobacteriaceae bacteria. It will be understood that those skilled in the art can replace the above-described *E. coli* strains with other expression strains as host cells.

The present disclosure also provides a nucleic acid molecule comprising a nucleic acid sequence encoding a collagen of the present disclosure. The nucleic acid may be DNA or cDNA. The nucleic acid molecule may consist mainly of the nucleic acid sequence encoding the peptide of the present disclosure, or may consist solely of the nucleic acid sequence encoding the peptide of the present disclosure. Such nucleic acid molecules can be synthesized using methods known in the art. Due to the degeneracy of the genetic code, it will be understood by those skilled in the art that nucleic acid molecules of different nucleic acid sequences may encode the same amino acid sequences.

The present disclosure also provides a vector comprising the nucleic acid sequence of the present disclosure. Suitable vectors are known in the art for vector construction, including the selection of promoters and other regulatory elements, such as enhancer elements. The vectors described in the present disclosure include the sequences suitable for introduction into cells. For example, the vector may be an expression vector, in which the coding sequence of the collagen is controlled by its own cis-acting regulatory elements. The design of a vector facilitates gene integration or gene replacement in the host cell.

It will be understood by those of ordinary skill in the art that in the present disclosure, the term "vector" includes a DNA molecule, for example, a plasmid, a phage, a virus or other vector, which contains one or more heterologous or recombinant nucleic acid sequences. Suitable phage and viral vectors include, but are not limited to, lambda-phage, EMBL phage, simian virus, bovine wart virus, Epstein-Barr virus, adenovirus, herpes virus, mouse sarcoma virus, murine breast cancer virus, lentivirus, and the like.

The collagen of the present disclosure comprises the sequence set forth in SEQ ID NO. 1 or 3, or the sequence in which one or more amino acids are mutated (*e.g.*, substituted, deleted, inserted and/or added) in the sequence set forth in SEQ ID NO. 1 or 3, as long as the collagen of the present disclosure retains the therapeutic effect on a cardiovascular disease or an anti-vascular endothelial cell injury effect of the amino acid sequence of SEQ ID NO. 3. "Plurality " may be 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80.

Amino acid addition refers to the addition of an amino acid(s) at C-terminus or N-terminus of an amino acid sequence, such as SEQ ID NO. 1 or 3, as long as the collagen of the present disclosure retains the therapeutic effect on cardiovascular disease or anti-vascular endothelial cell injury effect of the amino acid sequence of SEQ ID NO. 3.

Amino acid substitution refers to the substitution of a certain amino acid residue(s) at a certain position(s) of an amino acid sequence, such as the sequence of SEQ ID NO. 1 or 3 with other amino acid residues, as long as the collagen of the present disclosure retains the therapeutic effect on cardiovascular disease or anti-vascular endothelial cell injury effect of the amino acid sequence of SEQ ID NO. 3.

Amino acid insertion refers to the insertion of an amino acid residue(s) at an appropriate position(s) of an amino acid sequence, such as the sequence of SEQ ID NO. 1 or 3 and all or part of the inserted amino acid residues can be adjacent to one another, as long as the collagen of the present disclosure retains the therapeutic effect on cardiovascular disease or anti-vascular endothelial cell injury effect of the amino acid sequence of SEQ ID NO. 3. The insertion position of the amino acid herein is not located between the repeat sequences.

Amino acid deletion refers to that one, two, or three or more amino acids can be deleted from the amino acid sequence, such as the sequence of SEQ ID NO. 1 or 3, as long as the collagen of the present disclosure retains the therapeutic effect on cardiovascular disease or anti-vascular endothelial cell injury effect of the amino acid sequence of SEQ ID NO. 3.

In the present disclosure, the substitution may be a conservative amino acid substitution, which means that compared with the amino acid sequence of SEQ ID NO. 1 or 3, 3 amino acids, more preferably 2 or 1 amino acid, are replaced by amino acids with similar or related properties to form a peptide. In the context of the present disclosure, a conservative substitution may be defined as a substitution within one or more of the amino acid classes reflected in the following tables:

Conserved classes of amino acid residues:

| | |
|---|---|
| Acidic residues | D and E |
| Basic residues | K, R, and H |
| Hydrophilic uncharged residues | S, T, N and Q |
| Aliphatic uncharged residues | G, A, V, L and I |
| Non-polar uncharged residues | C, M and P |
| Aromatic residues | F, Y and W. |

Physical and functional classification of alternative amino acid residues:

| Alcohol-containing residues | S and T |
| Aliphatic residues | I, L, V and M |
| Cycloalkenyl-related residues | F, H, W and Y |
| Hydrophobic residues | A, C, F, G, H, I, L, M, R, T, V, W and Y |
| Negatively charged residues | D and E |
| Polar residues | C, D, E, H, K, N, Q, R, S and T |
| Positively charged residues | H, K and R |
| Small residues | A, C, D, G, N, P, S, T and V |
| Minimal residues | A, G and S |
| Residues involved in turn formation | A, C, D, E, G, H, K, N, Q, R, S, P and T |
| Flexible residues | Q, T, K, S, G, P, D, E and R. |

The degree of association between two amino acid sequences or between two nucleotide sequences is described by the parameter "sequence identity". For the purposes of the present disclosure, the Needleman-Wunsch algorithm (Needleman and Wunsch, 1970, J. Mol. Biol. 48: 443-453) as implemented by the needle program in the EMBOSS software package (EMBOSS: European Open Software Suite for Molecular Biology, Rice et al., 2000, Trends Genet. 16: 276-277) (preferably version 5.0.0 or later) is used to determine sequence identity between two amino acid sequences. The parameters used are a gap open penalty of 10, a gap extension penalty of 0.5, and an EBLOSUM62 (EMBOSS version of BLOSUM62) substitution matrix. The output of needle labeled "longest identity" (obtained using the non-simplification option) is used as percent identity and calculated as follows:

(identical residues $\times$ 100) / (length of alignment - total number of gaps in alignment)

For the purposes of the present disclosure, the Needleman-Wunsch algorithm (Needleman and Wunsch, 1970, *supra*) as implemented by the needle program in the EMBOSS software package (EMBOSS: European Open Software Suite for Molecular Biology, Rice et al., 2000, *supra*) (preferably version 5.0.0 or later) is used to determine sequence identity between two deoxynucleotide sequences. The parameters used are a gap open penalty of 10, a gap extension penalty of 0.5, and an EDNAFULL (EMBOSS version of NCBI NUC4.4) substitution matrix. The output of needle labeled "longest identity" (obtained using non-simplification option) is used as percent identity and calculated as follows:

(identical deoxynucleotides 100) / (length of alignment - total number of gaps in alignment)

The term "subject" refers to any human or other animal, particularly other mammals, receiving prevention, treatment, or diagnosis. Other mammals may include, for example, dog, cat, cow, horse, sheep, pig, goat, rabbit, rat, guinea pig, mouse, and the like. In some embodiments, the subject includes humans of any age with cardiovascular disease risk factors. Common risk factors include age, sex, weight, family history, sleep apnea, alcohol drinking or smoking, lack of exercise, induced arrhythmia, or signs of insulin resistance.

The term "treatment" refers to the medical management of a patient intended to cure, improve, stabilize, or prevent a disease, pathological condition, or disorder. The term includes active treatment, i.e., treatment specifically intended to improve a disease, pathological condition or disorder, and also includes causal treatment, i.e., treatment intended to remove the cause of the related disease, pathological condition or disorder. Furthermore, the term includes palliative treatment, i.e., treatment designed to alleviate symptoms rather than cure a disease, pathological condition or disorder; prophylactic treatment, i.e., treatment intended to minimize or partially or completely inhibit the development of the related disease, pathological condition or disorder; and supportive treatment, i.e., treatment used to supplement another specific therapy intended to ameliorate the related disease, pathological condition or disorder. In some embodiments, the recombinant collagen of the present disclosure can treat a cardiovascular disease, can be used for the repair of a vascular injury, or treat a vascular injury. In some embodiments, the recombinant collagen of the present disclosure can prevent (i.e., prophylactic treatment) a cardiovascular disease or a vascular injury. In some embodiments, the recombinant collagen of the present disclosure can be used as a supportive treatment. That is, the collagen of the present disclosure can be used to supplement the treatment of another specific therapy intended to ameliorate the related disease, pathological condition or disorder.

Ang II-induced human umbilical vein endothelial cell model

Vascular endothelial cell injury is an important cause of cardiovascular diseases such as hypertension, and its degree of injury is positively correlated with the severity of hypertension. Cell injury will lead to changes in secretory function of a cell, such as increased vascular permeability, increased vasoconstrictor factors, decreased vasodilation factors, resulting in increased vascular pressure and forming hypertension. Hypertension further causes endothelial cell injury, forming a vicious circle. Human umbilical vein endothelial cells (HUVECs) share similar biological characteristics with vascular endothelial cells, are close to the physiological state of the human body, and have no species differences. They are easy to obtain, rich in sources, and ethically compliant, thus being widely used by researchers at home and abroad to establish cell models simulating hypertensive injury. Guoyan Liu et al. (Food Science, 174, vol.38, No.13, 2017) have used Ang II-induced human umbilical vein endothelial cells to establish a hypertensive injury model.

Treatment Methods

A method of preventing and/or treating a cardiovascular disease or for the repair of a vascular injury or for preventing and/or treating a vascular injury in a subject is provided herein, comprising administering to the subject the collagen, the fusion protein comprising the collagen, the nucleic acid encoding the collagen or the fusion protein, and/or the vector comprising the nucleic acid, or the composition comprising the collagen, the fusion protein, the nucleic acid, and/or the vector described herein. The collagen may be any of the collagens described in the first aspect herein. The subject can also have an ultraviolet photoaging-induced skin injury or a diabetic infected wound. Therefore, the method of the present disclosure is also used for preventing and/or treating the ultraviolet photoaging-induced skin injury or the diabetic infected wound. The vascular injury may be a vascular endothelial injury.

The cardiovascular disease is a cardiovascular disease associated with an endothelial cell injury, preferably, the endothelial cell injury is endothelial cell injury induced by angiotensin II, and/or the vascular injury is a vascular injury induced by angiotensin II. The cardiovascular disease may be selected from atherosclerosis, hypertension, coronary heart disease, peripheral vascular disease, stroke, heart failure, arrhythmia, cerebrovascular disease, atrial fibrillation, and thrombotic disease. The method of the present disclosure can also include administering to the subject a lipid-regulating agent and/or an antihypertensive agent, such as statins, an angiotensin converting enzyme inhibitor, a calcium channel blocker and/or a beta receptor blocker.

Use

Provided herein are use of a collagen, a fusion protein comprising the collagen, a nucleic acid encoding the collagen or the fusion protein, a vector comprising the nucleic acid, a host cell comprising the vector, or a composition comprising the collagen, the fusion protein, the nucleic acid, the vector and/or the host cell for the manufacture of a medical device or a medicament or a kit for preventing and/or treating a cardiovascular disease or for the repair of a vascular injury or for preventing and/or treating a vascular injury in a subject. The collagen may be any of the collagens described in the first aspect herein. The subject can also have an ultraviolet photoaging-induced skin injury or a diabetic infected wound. Therefore, the medical device or medicament is also used for preventing and/or treating an ultraviolet photoaging-induced skin injury or a diabetic infected wound. The vascular injury may be a vascular endothelial injury.

Provided herein are also a collagen, a fusion protein comprising the collagen, a nucleic acid encoding the collagen or the fusion protein, a vector comprising the nucleic acid, a host cell comprising the vector or a composition comprising the collagen, the fusion protein, the nucleic acid, the vector and/or the host cell for use in preventing and/or treating a cardiovascular disease or for use in the repair of a vascular injury or for use in preventing and/or treating a vascular injury in a subject. The vascular injury may be a vascular endothelial injury. The collagen may be any of the collagens described in the first aspect herein. The collagen, the fusion protein comprising the collagen, the nucleic acid encoding the collagen or the fusion protein, the vector comprising the nucleic acid, the host cell comprising the vector or the composition comprising the collagen, the fusion protein, the nucleic acid, the vector and/or the host cell can also be used for preventing and/or treating an ultraviolet photoaging-induced skin injury or a diabetic infected wound.

The cardiovascular disease is a cardiovascular disease associated with an endothelial cell injury, preferably, the endothelial cell injury is an endothelial cell injury induced by angiotensin II, and/or the vascular injury is a vascular injury induced by angiotensin II. The cardiovascular disease may be selected from atherosclerosis, hypertension, coronary heart disease, peripheral vascular disease, stroke, heart failure, arrhythmia, cerebrovascular disease, atrial fibrillation, and thrombotic disease. The method of the present disclosure can also include administering to the subject a lipid-regulating agent and/or an antihypertensive agent, such as statins, an angiotensin converting enzyme inhibitor, a calcium channel blocker and/or a beta receptor blocker.

*In vitro* methods

The present disclosure relates to a method of blocking or eliminating an angiotensin II-induced vascular endothelial cell injury in vitro, comprising the step of contacting the vascular endothelial cell with the collagen described herein, preferably, the vascular endothelial cell is an umbilical vein vascular endothelial cell, preferably a human umbilical vein vascular endothelial cell.

The present disclosure relates to *in vitro* methods for (1) reducing angiotensin II-induced ROS production in a cell, (2) alleviating microtubule injury, preferably Ang II-induced microtubule injury in a cell, comprising a step of contacting the cell with the collagen described herein, preferably, the cell is a vascular endothelial cell, preferably an umbilical vein vascular endothelial cell, preferably a human umbilical vein vascular endothelial cell.

Compositions

The composition of the present disclosure may be a pharmaceutical composition. The pharmaceutical composition may comprise a pharmaceutically acceptable carrier, such as an adjuvant, a preservative, a wetting agent, an emulsifying agent, and a dispersing agent. The absence of microorganisms can be ensured by the sterilization procedures described above and by the addition of various antibacterial and antifungal agents (such as parabens, chlorobutanol, phenol, sorbic acid). It may also be desirable to include an isotonic agent such as sugar, sodium chloride, and the like in the composition. Furthermore, prolonged absorption of the injectable medicament form can be achieved by including an agent that delays absorption (such as aluminum monostearate and gelatin). The pharmaceutically acceptable carrier is formulated according to a number of factors within the common knowledge of those skilled in the art. These include, but are not limited to, the type and nature of the active agent formulated; the subject to whom the composition containing the agent is administered; the intended route of administration of the pharmaceutical composition; and target therapeutic indications. The pharmaceutically acceptable carrier includes both aqueous and non-aqueous liquid media, as well as various solid and semi-solid dosage forms. Such carriers may also include many different ingredients and additives in addition to the active agent. Such additional ingredients are comprised in the formulation for a variety of reasons well known to those of ordinary skill in the art (such as stabilization of the active agent, binder, *etc*.). The description of suitable pharmaceutically acceptable carriers and factors involved in their selection can be found in a variety of readily available sources such as, for example, Allen, L.V., Jr. et al., Remington: The Science and Practice of Pharmacy (Vol. 2), 22nd ed., Pharmaceutical Press (2012).

The collagen or the pharmaceutical composition can be administered via an oral or parenteral route, including, but not limited to, intravenous, intramuscular, intraarterial, intrathecal, intracapsular, intraorbital, intracardiac, intradermal, intraperitoneal, intratracheal, subcutaneous, subepidermal, intraarticular, subcapsular, subarachnoid, intraspinal, epidural, and intrasternal injection and infusion.

Particularly when formulated as a single dosage unit, the active ingredients in the combination may undergo chemical interactions. For this reason, when the collagen of the present disclosure is combined with a second therapeutic agent in a single dosage unit, they are formulated to minimize (i.e., reduce) physical contact between the active ingredients despite their coexistence in the same single dosage unit. For example, the active ingredient may be enteric-coated. By enteric coating of one of the active ingredients, it can not only minimize the contact between the active ingredients of the combination, but also control the release of one of these components in the gastrointestinal tract, such that one of these components is not released in the stomach, but in the intestinal tract. One of the active ingredients can also be used as a coating material, and the material affects sustained-release throughout the gastrointestinal tract and can also be used to minimize the physical contact between the combined active ingredients. In some embodiments, in order to protect the collagen from digestion by gastric juice, the collagen can be coated.

Another method involves formulating a combination product, wherein one ingredient is coated with a sustained-release and/or enteric-release polymer and the other ingredient is also coated with a polymer (such as a low-viscosity grade hydroxypropyl methyl cellulose (HPMC)) or other suitable materials as known in the art to further separate the active components. The polymer coating is used to form an additional barrier against interaction with the other component.

The dosing regimen of the collagen of the present disclosure will vary depending on known factors (such as the pharmacodynamic profile of the particular agent and its mode and route of administration; the species, age, sex, health, medical condition and weight of the recipient; the nature and extent of symptoms; the kind of concurrent treatment; the frequency of treatment; the route of administration, the renal and liver function of the patient and the desired effect).

Medical device

"Medical device" refers to an instrument, a device, an appliance, an *in vitro* diagnostic reagent and a calibrator, a material, and other similar or related articles that are directly or indirectly used in the human body. The collagen described herein can serve as a medical device, and thus can be used as a medical device. In some embodiments, the collagen of the present disclosure exerts pharmacological effects and can be used as a medicament. In one embodiment, the collagen of the present disclosure is used as a drug-device combination product.

The collagen or recombinant collagen described herein is useful for a variety of purposes. In some embodiments, the collagen or recombinant collagen is directly applied to or introduced into the body of the subject, particularly the site of the vascular injury as a medical device. In some embodiments, the collagen or recombinant collagen is used as a medical device in a variety of therapeutic or prophylactic applications. Such applications can be used to prevent or treat a vascular injury, for example, in a vascular injury involving the skin and other collagen-containing structures and organs. The collagen or recombinant collagen described herein can also be used to provide biocompatible coatings for certain medical devices to promote healing of injuries and disorders in body areas in which such devices are used to treat or prevent cardiovascular disease in a subject.

The following examples are further provided to illustrate the present disclosure.

Examples

Hereinafter, the inventive objects, technical features, and beneficial effects of the present disclosure are further described in detail in conjunction with examples, but the embodiments of the present disclosure are not limited thereto.

The inventors performed cytotoxicity experiments, which verified that RhCOL III had no effect on the cell viability of human umbilical vein endothelial cells (HUVECs) within the concentration range of 0.1-2 mg/mL.

The inventors then evaluated the protective effect of RhCOL III on angiotensin II (AngII)-induced endothelial injury in HUVECs. The results showed that RhCOL III inhibited the fluorescence intensity of intracellular reactive oxygen species (ROS).

RhCOL III used herein was prepared and obtained by the inventors. The sequence was GERGAPGFRGPA GPNGIPGEKGPAGERGAPGERGAPGFRGPAGPNGIPGEKGPAGERG APGERGAPGFRGPAGPNGIPGEKGPAGE RGAPGERGAPGFRGPAGPNGIPGEKGPAGE RGAPGERGAPGFRGPAGPNGIPGEKGPAGERGAPGERGAPGFRG PAGPNGIPGEKGPA GERGAPGERGAPGFRGPAGPNGIPGEKGPAGERGAPGERGAPGFRGPAGPNGIPGEKG PA GERGAPGERGAPGFRGPAGPNGIPGEKGPAGERGAPGERGAPGFRGPAGPNGIPGE KGPAGERGAPGERGAPGF RGPAGPNGIPGEKGPAGERGAPGERGAPGFRGPAGPNGIP GEKGPAGERGAPGERGAPGFRGPAGPNGIPGEKG PAGERGAPGERGAPGFRGPAGPN GIPGEKGPAGERGAPGERGAPGFRGPAGPNGIPGEKGPAGERGAPGERGAP GFRGPAG PNGIPGEKGPAGERGAP. The specific preparation process can be found in CN 201811438582.6.

Example 1: RhCOL III Functions by attaching to the cell surface.

Recombinant collagen RhCOL III solution was formulated with 0.1 M sodium carbonate buffer (pH 9.0), with a concentration of ≥ 2 mg/mL and a volume of 20 mL. FITC solution (manufacturer: MCE, product catalog no.: HY-66019) was added to the recombinant collagen solution in aliquots of 5 μL at a time with multiple additions while the mixture was gently stirred, at a ratio of 50 μL FITC solution per 1 mL of recombinant collagen solution. After the required FITC addition was complete, the reaction solution was incubated at 4 °C in the dark for 8 h. 1.05 mL of $NH_4Cl$ solution was added to the reaction solution, followed by gentle shaking to adjust the final concentration of $NH_4Cl$ to 50 mM; the reaction was terminated by incubation at 4 °C for 2 h, and subsequent dialysis was performed overnight. HUVECs were inoculated into a 24-well plate at the density of $2 \times 10^4$, the prepared recombinant collagen labeled with FITC was added upon adherence and incubated in the dark for 30 min, then fixed with 4% paraformaldehyde for 10 min. After adding antifade mounting medium, the cells were observed and photographed using a fluorescence inverted microscope.

Experimental results:

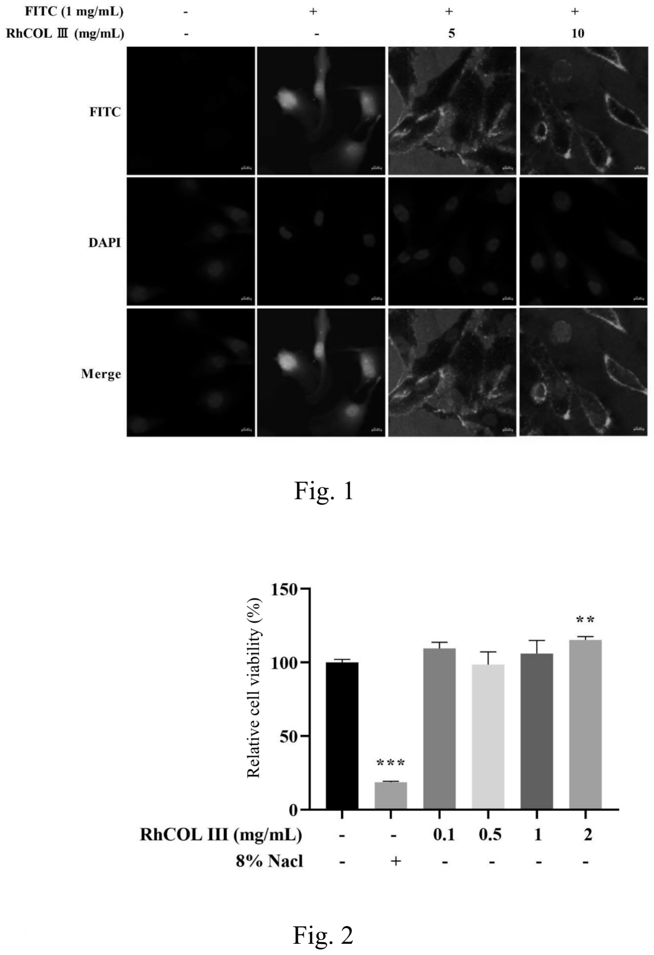

In order to study the interaction mechanism between RhCOL III and HUVEC, we utilized FITC to label RhCOL III. It could be seen from Fig. 1 that a large number of RhCOL III collagen attached to the surface of HUVEC cells, indicating that RhCOL III did not enter the cells but exerted its function by attaching to the cell surface.

Example 2: Determination of the effect of RhCOL III on HUVEC cells by CCK8 assay

HUVEC cells were cultured in an incubator containing 5% carbon dioxide at the temperature of 37 °C using ECM medium (manufacturer: Sciencell, product cat. No.: 1001) (1% penicillin-streptomycin, 5% fetal bovine serum). $5 \times 10^3$ cells were uniformly plated in 96-well plates and cultured in an incubator containing 5% carbon dioxide at the temperature of 37 °C. Starvation treatment was performed for 12 h upon adherence, then RhCOL III (dissolved in ECM medium) at different concentrations (0.1, 0.5, 1 and 2 mg/mL) or 8% NaCl (used as a control) was added and incubated for 24 h. After washing, 100 μL of 1 mg/mL CCK-8 (manufacturer: Vazyme, product cat. No.: A311-01) was added and incubated for 1 h. The absorbance was determined at 450 nm with a microplate reader. The calculation formula of relative cell viability is:

$$\text{Cell viability (\%)} = (\text{measured value - blank value}) / (\text{control value - blank value}) \times 100\%$$

Experimental results:

The effects of the addition of RhCOL III at different concentrations (0.1, 0.5, 1 and 2 mg/mL) on the viability of HUVEC cells were detected, and the results were shown in Fig. 2. The results showed that the sample did not affect the viability of HUVEC cells at concentrations below 2 mg/mL, which could be used in subsequent experiments.

Example 3: Determination of intracellular reactive oxygen species (ROS)

Abnormal vascular function is the key feature of hypertension, atherosclerosis, diabetes, and aging, and thus is the major contributing factor to morbidity and mortality. A common factor driving vascular dysfunction in these disease states is overproduction of reactive oxygen species (ROS). Superoxide anions and associated ROS not only quench nitric oxide (NO), but also directly impair the function of cellular proteins through oxidative post-translational modifications, thereby driving inflammation, cell proliferation, fibrosis, atherosclerosis, and damage to membrane transport. The main contributing factor to this elevated ROS in vascular diseases is activation of the renin-angiotensin system, which leads to angiotensin II (Ang II)-mediated activation of NADPH oxidase, as well as uncoupling of endothelial nitric oxide synthase (eNOS).

$2 \times 10^4$ HUVEC cells were uniformly plated into a 24-well plate and cultured in ECM medium in an incubator with 5% carbon dioxide at 37 °C. Upon adherence, the cells were subjected to starvation treatment for 12 h. The old medium in the 24-well plate was discarded, and RhCOL III (formulated in ECM medium) at different concentrations (0.1, 0.5, 1, and 2 mg/mL) was added, followed by incubation for 2 h. Subsequently, 1 $\mu$M Ang II (formulated in ECM medium) was added to each well, and the cells were incubated for 24 h. The old medium was discarded, and the cells were carefully washed with PBS buffer three times. Then, 200 $\mu$L of 10 $\mu$M DCFH-DA reagent (manufacturer: Beyotime, product catalog no.: S0033M) was added, and the cells were incubated in a 37 °C constant temperature incubator for 30 min. After incubation, the cells were carefully washed with PBS buffer three times. Throughout the experiment, wells without RhCOL III and Ang II treatment served as the blank group. The cells were placed under an inverted fluorescence microscope for observation and photography. The average optical density was calculated using ImageJ software.

Experimental results:

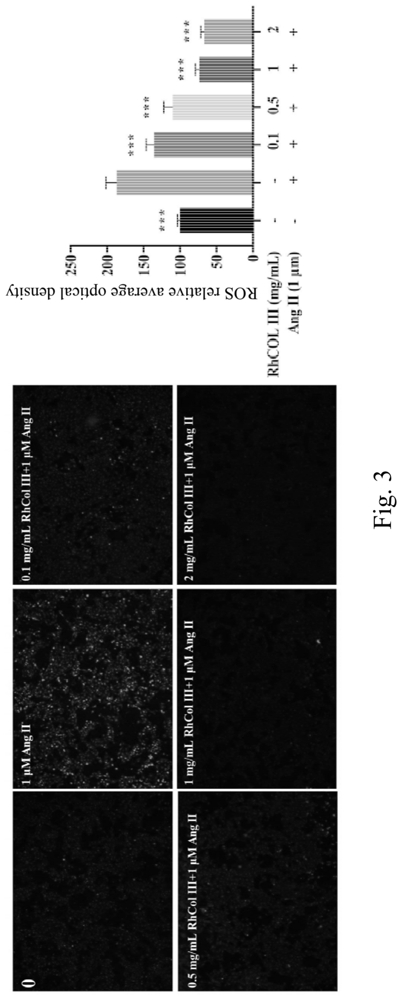

The effects of RhCOL III at different concentrations (0.1, 0.5, 1, and 2 mg/mL) on ROS production in HUVEC cells were detected. As shown in Fig. 3, the blank group exhibited no obvious fluorescence, while the Ang II-treated group showed a significant increase in ROS fluorescence intensity compared with the blank group. RhCOL III treatment attenuated the Ang II-induced increase in ROS production. RhCOL III significantly inhibited ROS production in HUVECs in a dose-dependent manner. Ang II binds to the angiotensin type I receptor (AT1-R) to activate NADPH oxidase, promote ROS production, and enhance oxidative stress, a common mechanism underlying hyperlipidemia, hypertension, diabetes, and atherosclerosis (Chen, L. Y., Wu, Y. Q., Zhang, Z. H., Luo, Q. P., Wang, Z. C., et al. The NADPH redox reaction platform and its regulatory role in the Ang II-mediated ROS signaling pathway. Progress in Physiological Sciences, 2012(6), 41-46.). Treatment with RhCOL III in HUVECs may abrogate Ang II-induced ROS production and alleviate Ang II-mediated microtubule injury. Collectively, these results suggest that RhCOL III has potential applications in the treatment or prevention of vascular injury and cardiovascular diseases.

Example 4: RhCOL III treatment could significantly alleviate microtubule injury induced by Ang II.

HUVEC cells were uniformly plated into a 24-well plate, and starvation treatment was performed for 12 h upon adherence. The old medium in the 24-well plate was discarded. RhCOL III at different concentrations (0.1, 0.5, 1 and 2 mg/mL) was added and the cells were incubated for 2 h. Then, 1 $\mu$M of Ang II was added to each well and the cells were incubated for 24 h. The old medium was discarded. The cells were carefully washed with PBS buffer solution for 3 times. After fixing with 4% formaldehyde solution at room temperature for 15 min, PBS containing 0.1% Triton X-100 was added and the cells were incubated for 10 min. Finally, Tubulin-Tracker Red staining working solution (manufacturer: Beyotime, product cat.no. C1050) was added and the cells were incubated at room temperature in the dark for 30-60 min. The cells were carefully washed with PBS buffer solution for 3 times. Antifade mounting medium containing DAPI was added and the cells were placed under a fluorescent inverted microscope for observation and photography.

Experimental results:

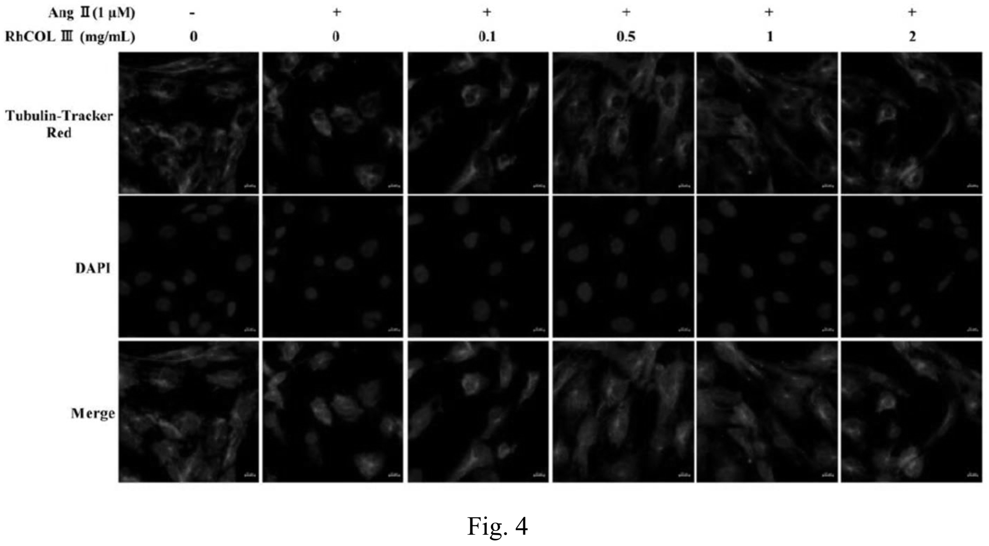

The effects of the addition of RhCOL III at different concentrations (0.1, 0.5, 1 and 2 mg/mL) on microtubule formation in HUVEC cells were detected, as shown in Fig. 4, the Ang II group inhibited microtubule formation compared with the control group. RhCOL III treatment could significantly alleviate Ang II-induced microtubule injury.

Example 5: Monitoring blood pressure, heart rate, and body weight in spontaneously hypertensive rats

Male spontaneously hypertensive rats (SHRs, 8 weeks old) were purchased from Beijing Vital River Laboratory Animal Technology Co., Ltd. (Beijing, China). SHRs were randomly divided into SHR group, captopril treatment group (10 mg/kg/day), and RhCOL III treatment group (10 mg/kg/day), with 6 rats per group. All animals were maintained on a 12-h light-dark cycle (12 h light/12 h dark) under specific pathogen-free (SPF) conditions with a particular constant temperature (20 $\pm$ 26 °C) and relative humidity (40-70%). The blood pressure of rats in each group was measured using a non-invasive tail artery sphygmomanometer once weekly for 4 consecutive weeks. First, the non-invasive tail artery sphygmomanometer was preheated and calibrated for performance. Then, each rat was placed in a quiet environment to acclimatize to the surroundings. The base of the tail was positioned on the sensor of the sphygmomanometer, aligned with the tail artery, and the measurement button was pressed. The instrument automatically measured the systolic blood pressure, heart rate, and body weight of the rats.

Experimental results:

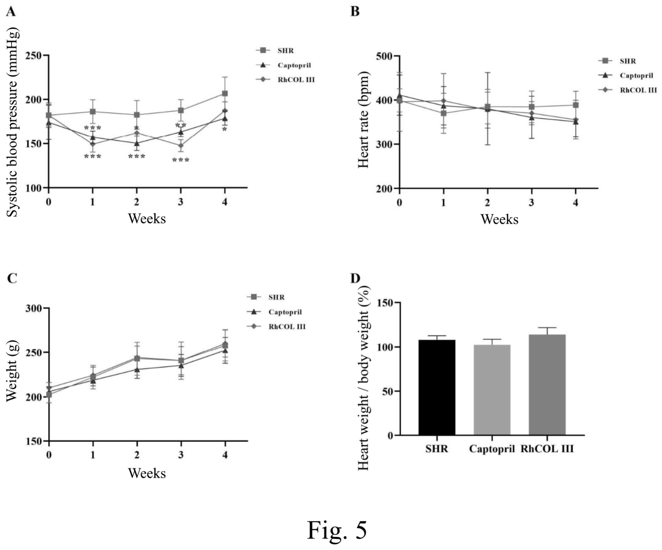

To evaluate the antihypertensive effect of RhCOL III on SHRs, we measured the tail artery blood pressure of SHRs. As shown in Fig. 5, RhCOL III treatment may significantly alleviate the elevated blood pressure. We also evaluated the effects of RhCOL III on body weight, heart rate, and heart weight ratio of SHRs, and the results showed that RhCOL **III** had no effect on body weight, heart rate, and heart weight ratio. Compared with the control group (SHR group), the RhCOL III treatment group and the captopril treatment group had reduced systolic blood pressure. These results revealed that RhCOL III could reduce blood pressure in spontaneously hypertensive rats.

Example 6: Biochemical determination of blood samples from spontaneously hypertensive rats

For each treatment group of Example 5, orbital blood sampling was performed after 4 weeks of continuous injection. The whole blood samples were allowed to stand overnight at 4 °C, then centrifuged at 3000 rpm for 15 min (2-8 °C). The supernatant was collected for immediate analysis, and the content of each indicator was determined according to the instructions of the kit (the manufacturer and product catalog number of each kit are shown in the table below).

Table 1 Kit Information

| Kit Name | Catalog No.: | Manufacturer |
| --- | --- | --- |
| Lactic Acid Assay Kit | A019-2-1 | Nanjing JianCheng |
| Alanine Aminotransferase ALT Assay Kit | S03030 | Rayto |
| Uric Acid Assay Kit | S03035 | Rayto |
| Creatinine Assay Kit | S03076 | Rayto |
| Lactate Dehydrogenase Assay Kit | S03034 | Rayto |

Experimental results:

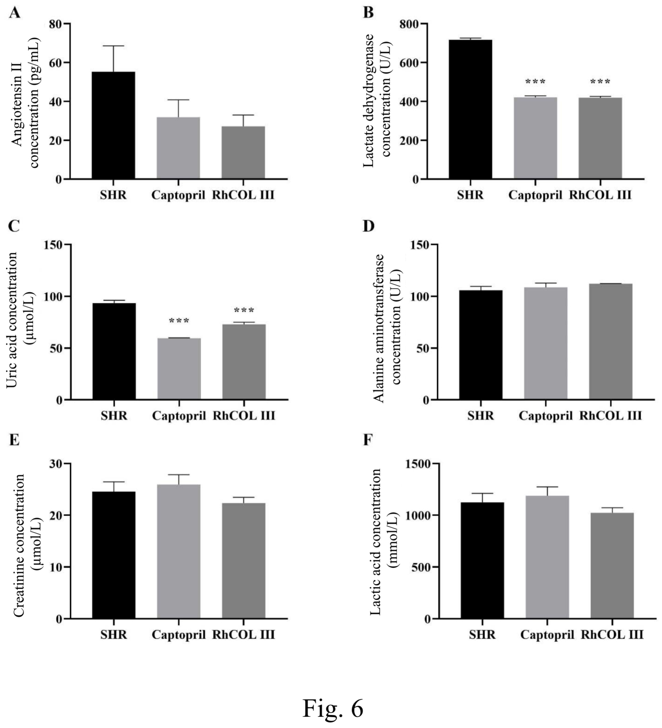

In order to further study the effect of RhCOL III on SHRs, we determined a series of biochemical indicators. As shown in plots B-F in Fig. 6, RhCOL III can significantly reduce the levels of lactate dehydrogenase and uric acid, effectively alleviating kidney injury, but had no significant effect on the levels of alanine aminotransferase, creatinine and lactate.

Example 7: Determination of plasma Ang II concentration

For each treatment group of Example 6, orbital blood sampling was performed after 4 weeks of continuous injection. 200 μL of plasma sample to be tested was taken respectively, and 200 μL of incubation solution was added (weighed 7.40 g of EDTA, 12.11 g of Tris-base, 0.063 g of PMSF and 0.01 mg of SBTI, dissolved in 90 mL of distilled water, and adjusted pH to 5.4-5.5 with acetic acid). After incubating at 37 °C for 3 h, 300 μL of Ang II internal standard working solution (stock solution diluted with 20% acetonitrile) was added to obtain pretreated samples. An SPE plate (Oasis MAX μElution SPE, Waters, USA) was activated with 200 μL of 50% acetonitrile aqueous solution containing 1% formic acid on a 96-well positive pressure device (Oasis MAX μElution SPE, Waters, USA). 550 μL of pretreated sample was taken onto the SPE plate, then 200 μL of 10% methanol containing 1% ammonia was added to wash impurities. Subsequently, the waste liquid plate below the SPE plate was replaced with a 96-well sample plate. The test substance was eluted with 40 μL

of 50% acetonitrile aqueous solution containing 1% formic acid, collected into a sample plate, and then analyzed by LC-MS/MS. Linearity was evaluated using a multiple regression equation, and the linear equation and correlation coefficient (R) were recorded for the quantification of Ang II in the samples.

Experimental results:

Ang II (angiotensin II) is the main bioactive peptide in the renin-angiotensin-aldosterone system (RAAS) and is the main cause of hypertension. As shown in panel A of Fig. 6, RhCOL III can significantly reduce the level of Ang II.

The above-described examples are preferred embodiments of the present disclosure, but the embodiments of the present disclosure are not limited to the above-described examples. Any other changes, modifications, substitutions, combinations, and simplifications made without departing from the spirit and essence of the present disclosure shall be equivalent replacement methods and are included within the scope of protection of the present disclosure.

## Claims

**1.** Use of a collagen, a fusion protein comprising the collagen, a nucleic acid encoding the collagen or the fusion protein, a vector comprising the nucleic acid, a host cell comprising the vector, or a composition comprising the collagen, the fusion protein, the nucleic acid, the vector and/or the host cell for the manufacture of a medical device or a medicament or a drug-device combination product or a kit for preventing and/or treating a cardiovascular disease, for repairing a vascular injury, or for preventing and/or treating a vascular injury in a subject, wherein the collagen comprises n repeat units and wherein the repeat unit comprises:

(1) a sequence set forth in SEQ ID NO. 1;
(2) a sequence having at least 80%, at least 85%, at least 90%, at least 91%, at least 92%, at least 93%, at least 94%, at least 95%, at least 96%, at least 97%, at least 98%, or at least 99% identity to the sequence set forth in SEQ ID NO. 1; or
(3) a sequence in which a substitution, an addition, a deletion and/or an insertion of 1 to 5 amino acid residues is introduced to the sequence set forth in SEQ ID NO. 1;
wherein n is an integer greater than or equal to 1, preferably an integer ranging from 1 to 32, such as 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31, wherein when n is an integer greater than or equal to 2, the repeat sequences are directly linked to each other;
preferably, the subject has an ultraviolet photoaging-induced skin injury or a diabetic infected wound;
preferably, the collagen has an anti-endothelial cell injury effect;
preferably, the vascular injury is a vascular endothelial injury;
preferably, the medical device or the medicament or the drug-device combination product or the kit is further capable of preventing and/or treating the ultraviolet photoaging-induced skin injury or the diabetic infected wound;
preferably, the collagen is a recombinant type III humanized collagen, preferably 164.88-degree recombinant type III humanized collagen; preferably, the recombinant type III humanized collagen is in a trimeric form;
preferably, the fusion protein comprises the collagen and a protein for promoting secretion, isolation and/or purification of the collagen;
preferably, the protein is selected from an enzyme cleavage site sequence, a signal peptide and a purification tag sequence;
preferably, the enzyme cleavage site sequence is a TEV protease cleavage site sequence;
preferably, the protein tag sequence is His tag, GST tag, MBP tag, SUMO tag, Cytiva Protein Select tag or NusA tag;
preferably, the collagen is linked to the protein directly or through a linker, wherein the linker is a flexible linker, such as $(G)_a$ or $(GGGGS)_b$, wherein a and b are each independently an integer from 1 to 10 or from 1 to 5 or from 1 to 3;
preferably, the medical device is a gel, a dressing, an invasive device, or an implantable device.

**2.** The use according to claim 1, wherein the collagen comprises the sequence set forth in SEQ ID NO. 2 or does not comprise the sequence set forth in SEQ ID NO. 2.

**3.** The use according to claim 1 or 2, wherein the collagen comprises:

a) an amino acid sequence of SEQ ID NO. 3;

b) an amino acid sequence which has at least 80%, at least 85%, at least 90%, at least 91%, at least 92%, at least 93%, at least 94%, at least 95%, at least 96%, at least 97%, at least 98%, or at least 99% identity to the amino acid sequence of SEQ ID NO. 3 while retaining the anti-endothelial cell injury effect of the amino acid sequence of SEQ ID NO. 3; or

c) an amino acid sequence in which 1 to 80, such as 1 to 70, 1 to 60, 1 to 50, 1 to 40, 1 to 30, 1 to 10, 1 to 8 or 1 to 5 amino acid residues are added, substituted, deleted or inserted into the amino acid sequence of SEQ ID NO. 3 while retaining the anti-endothelial cell injury effect of the amino acid sequence of SEQ ID NO. 3.

4. The use according to any one of claims 1-3, wherein the cardiovascular disease is a cardiovascular disease associated with an endothelial cell injury, preferably, the endothelial cell injury is an endothelial cell injury induced by angiotensin II or ROS, and/or the vascular injury is a vascular injury induced by angiotensin II or ROS.

5. The use according to any one of claims 1-4, wherein the cardiovascular disease is selected from arteriosclerosis, atherosclerosis, hypertension, coronary heart disease, peripheral vascular disease, stroke, heart failure, arrhythmia, cerebrovascular disease, atrial fibrillation, and thrombotic disease; preferably, wherein the hypertension is spontaneous hypertension; preferably, the medicament is a medicament for lowering blood pressure; preferably, the vascular endothelial injury is diabetic vascular endothelial injury.

6. The use according to any one of claims 1-5, wherein the composition comprises a lipid-regulating agent and/or an antihypertensive agent, such as statins, an angiotensin converting enzyme inhibitor, a calcium channel blocker and/or a beta receptor blocker.

7. A pharmaceutical composition comprising the collagen, the fusion protein comprising the collagen, the nucleic acid encoding the collagen or the fusion protein, the vector comprising the nucleic acid, the host cell comprising the vector in any one of claims 1 to 3, and an agent for preventing and/or treating a cardiovascular disease or for the repair of a vascular injury, preferably a lipid-regulating agent and/or an antihypertensive agent, such as statins, an angiotensin converting enzyme inhibitor, a calcium channel blocker and/or a beta receptor blocker.

8. A medical device comprising the collagen, the fusion protein comprising the collagen, the nucleic acid encoding the collagen or the fusion protein, the vector comprising the nucleic acid, the host cell comprising the vector in any one of claims 1-3, and a material or device for the repair of a vascular injury; wherein preferably, the medical device is a gel, a dressing, an invasive device, or an implantable device.

9. A method of blocking or eliminating an angiotensin II or ROS-induced vascular endothelial cell injury *in vitro*, comprising a step of contacting a vascular endothelial cell with the collagen in any one of claims 1 to 3, wherein preferably, the vascular endothelial cell is an umbilical vein vascular endothelial cell, preferably a human umbilical vein vascular endothelial cell.

10. An *in vitro* method for (1) reducing angiotensin II-induced ROS production in a cell or (2) alleviating microtubule injury, preferably Ang II-induced microtubule injury in a cell, comprising a step of contacting the cell with the collagen in any one of claims 1 to 3, wherein preferably, the cell is a vascular endothelial cell, preferably an umbilical vein vascular endothelial cell, preferably a human umbilical vein vascular endothelial cell.

11. A method of screening for an active ingredient for preventing and/or treating a cardiovascular disease or for the repair of a vascular injury in a subject, comprising

    (i) providing a collagen comprising n repeat units, wherein the repeating unit comprises:

        (1) a sequence set forth in SEQ ID NO. 1;
        (2) a sequence having at least 60%, at least 70%, at least 80%, at least 85%, at least 90%, at least 91%, at least 92%, at least 93%, at least 94%, at least 95%, at least 96%, at least 97%, at least 98%, or at least 99% identity to the sequence set forth in SEQ ID NO. 1; or
        (3) a sequence in which a substitution, an addition, a deletion and/or an insertion of 1 to 5 amino acid residues is introduced to the sequence set forth in SEQ ID NO. 1;

    wherein n is an integer greater than or equal to 1, preferably an integer ranging from 1 to 32, such as 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31, wherein when n is an integer greater than or equal to 2, the repeat sequences are directly linked to each other;

(ii) adding the collagen to an angiotensin II-treated HUVEC cell;

(iii) selecting a collagen having a property of: (a) reducing ROS generation/or (b) alleviating a microtubule injury, as an active ingredient;

preferably, the subject has an ultraviolet photoaging-induced skin injury or a diabetic infected wound;

preferably, the collagen has an anti-endothelial cell injury effect;

preferably, the active ingredient is further capable of preventing and/or treating the ultraviolet photoaging-induced skin injury or the diabetic infected wound;

preferably, wherein the cardiovascular disease is a cardiovascular disease associated with an endothelial cell injury, preferably, the endothelial cell injury is an endothelial cell injury induced by angiotensin II or ROS, and/or the vascular injury is a vascular injury induced by angiotensin II or ROS;

preferably, wherein the cardiovascular disease is selected from atherosclerosis, hypertension, coronary heart disease, peripheral vascular disease, stroke, heart failure, arrhythmia, cerebrovascular disease, atrial fibrillation, and thrombotic disease;

preferably, the selected collagen comprises:

a) the amino acid sequence of SEQ ID NO. 3;

b) an amino acid sequence having at least 80%, at least 85%, at least 90%, at least 91%, at least 92%, at least 93%, at least 94%, at least 95%, at least 96%, at least 97%, at least 98%, or at least 99% identity to the amino acid sequence set forth in SEQ ID NO. 3; or

c) an amino acid sequence in which 1 to 80, such as 1 to 70, 1 to 60, 1 to 50, 1 to 40, 1 to 30, 1 to 10, 1 to 8 or 1 to 5 amino acid residues are added, substituted, deleted or inserted into the amino acid sequence of SEQ ID NO. 3;

preferably, the active ingredient is useful as a medicament or as a medical device or as a drug-device combination product.

| FITC (1 mg/mL) | - | + | + | + |
|---|---|---|---|---|
| RhCOL Ⅲ (mg/mL) | - | - | 5 | 10 |

Fig. 1

Fig. 2

Fig. 3

Fig. 4

Fig. 5

Fig. 6

## INTERNATIONAL SEARCH REPORT

| International application No. |
| --- |
| **PCT/CN2024/104947** |

**A. CLASSIFICATION OF SUBJECT MATTER**

A61K38/39(2006.01)i; C07K14/78(2006.01)i; A61P9/00(2006.01)i; A61P9/12(2006.01)i; A61K8/65(2006.01)i; A61P9/12(2006.01)i

According to International Patent Classification (IPC) or to both national classification and IPC

**B. FIELDS SEARCHED**

Minimum documentation searched (classification system followed by classification symbols)

IPC: A61K, C07K, A61P

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, Web of Science, 百度学术, BAIDU SCHOLAR, Patentics, 中国生物序列检索系统, China Patents Biological Sequence Search System, NCBI Genbank, EBI, STNext: 申请人, 发明人, collagen, 胶原蛋白, 序列1-3, 高血压, 血管, 损伤, 修复, 心血管, ROS, hypertension, vascular, damage, repair, cardiovascular

**C. DOCUMENTS CONSIDERED TO BE RELEVANT**

| Category* | Citation of document, with indication, where appropriate, of the relevant passages | Relevant to claim No. |
| --- | --- | --- |
| Y | CN 109593126 A (SHANXI JINBO BIO-PHARMACEUTICAL CO., LTD.) 09 April 2019 (2019-04-09) <br> claims 1-9 | 1-11 |
| Y | "保健与生活" 杂志社编 ("Health & Life" Magazine Publisher). "鸡汤让你的血压"一路下降" (Non-official translation: Chicken Soup Keeps Your Blood Pressure Down)" <br> 保健与生活 *(Health & Life)*, 31 December 2012 (2012-12-31), <br> page 181 | 1-11 |
| Y | 洪惠等 (HONG, Hui et al.). "缓解氧化应激 (Non-official translation: Relieve Oxidative Stress)" <br> 胶原蛋白与胶原蛋白肽功能与应用 *(Collagen and Collagen Peptides Function and Application)*, 31 December 2022 (2022-12-31), <br> page 104 | 1-11 |
| Y | CN 104087641 A (TIANJIN UNIVERSITY) 08 October 2014 (2014-10-08) <br> abstract | 1-11 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **15 October 2024** | **21 October 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

# EP 4 717 271 A1

## INTERNATIONAL SEARCH REPORT

International application No.

**PCT/CN2024/104947**

### C. DOCUMENTS CONSIDERED TO BE RELEVANT

| Category* | Citation of document, with indication, where appropriate, of the relevant passages | Relevant to claim No. |
|---|---|---|
| Y | CN 109730188 A (TIBET YANGJIN ECOLOGICAL AGRICULTURE AND ANIMAL HUSBANDRY TECHNOLOGY CO., LTD.) 10 May 2019 (2019-05-10) abstract | 1-11 |
| Y | CN 113476593 A (SHANXI JINBO BIO-PHARMACEUTICAL CO., LTD.) 08 October 2021 (2021-10-08) claims 1-10 | 1-11 |
| Y | CN 114214384 A (HAINAN TERNARY SATELLITE BIOTECHNOLOGY CO., LTD.) 22 March 2022 (2022-03-22) abstract | 1-11 |
| A | EP 3985019 A1 (NANTONG UNIVERSITY) 20 April 2022 (2022-04-20) abstract | 1-11 |

Form PCT/ISA/210 (second sheet) (July 2022)

## INTERNATIONAL SEARCH REPORT

International application No.

**PCT/CN2024/104947**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
Information on patent family members

International application No.

**PCT/CN2024/104947**

| Patent document cited in search report | | | Publication date (day/month/year) | Patent family member(s) | | | Publication date (day/month/year) |
|---|---|---|---|---|---|---|---|
| CN | 109593126 | A | 09 April 2019 | EP | 3660045 | A1 | 03 June 2020 |
| | | | | EP | 3660045 | B1 | 07 August 2024 |
| | | | | US | 2020165319 | A1 | 28 May 2020 |
| | | | | US | 11396537 | B2 | 26 July 2022 |
| | | | | LT | 3660045 | T | 10 September 2024 |
| | | | | FI | 3660045 | T3 | 23 September 2024 |
| | | | | DK | 3660045 | T3 | 09 September 2024 |
| CN | 104087641 | A | 08 October 2014 | None | | | |
| CN | 109730188 | A | 10 May 2019 | None | | | |
| CN | 113476593 | A | 08 October 2021 | None | | | |
| CN | 114214384 | A | 22 March 2022 | None | | | |
| EP | 3985019 | A1 | 20 April 2022 | EP | 3985019 | A4 | 30 November 2022 |
| | | | | EP | 3985019 | B1 | 18 September 2024 |
| | | | | JP | 2022538087 | A | 31 August 2022 |
| | | | | JP | 7261511 | B2 | 20 April 2023 |
| | | | | WO | 2021212627 | A1 | 28 October 2021 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310890232

- CN 201811438582

**Non-patent literature cited in the description**

- **HUA C** ; **ZHU Y** ; **XU W** ; **YE S** ; **ZHANG R** ; **LU L** ; **JIANG S.** Characterization by high-resolution crystal structure analysis of a triple-helix region of human collagen type III with potent cell adhesion activity.. *Biochem Biophys Res Commun*, 22 January 2019, vol. 508 (4), 1018-1023
- **NEEDLEMAN** ; **WUNSCH**. *J. Mol. Biol.*, 1970, vol. 48, 443-453
- **RICE et al.** EMBOSS: European Open Software Suite for Molecular Biology. *Trends Genet.,* 2000, vol. 16

- **RICE et al.** *EMBOSS: European Open Software Suite for Molecular Biology*, 2000
- **GUOYAN LIU et al.** *Food Science*, 2017, vol. 38 (13)
- **ALLEN, L.V., JR. et al.** Remington: The Science and Practice of Pharmacy. Pharmaceutical Press, 2012, vol. 2
- **CHEN, L. Y.** ; **WU, Y. Q.** ; **ZHANG, Z. H** ; **LUO, Q. P** ; **WANG, Z. C. et al.** The NADPH redox reaction platform and its regulatory role in the Ang II-mediated ROS signaling pathway.. *Progress in Physiological Sciences*, 2012 (6), 41-46